# EUROPEAN PATENT APPLICATION

(11) **EP 4 748 384 A1**
(43) Date of publication of application: **27.05.2026**
(21) Application number: 24383261.5
(22) Date of filing: 21.11.2024
(51) Int. Cl.: A61K 31/727, A61K 31/737, A61P 39/00, A61K 31/728

(54) **GLYCOSAMINOGLYCAN, OR PHARMACEUTICAL COMPOSITION COMPRISING THEREOF, FOR USE IN A METHOD FOR THE PREVENTION AND/OR TREATMENT OF ANAPHYLAXIS**

(71) Applicant: Fundacion Instituto De Investigacion Sanitaria Fundacion Jimenez Diaz, 28040 Madrid (ES)
(72) Inventor: ESTEBAN VÁZQUEZ, Vanesa, 28040 Madrid (ES); MENDEZ BARBERO, Nerea, 28040 Madrid (ES)
(74) Representative: Hoffmann Eitle

(57) **Abstract**

The present invention refers to the medical field. Particularly, it refers to Glycosaminoglycan, or pharmaceutical composition comprising thereof, for use in a method for the prevention and/or treatment of anaphylaxis.

## Description

### FIELD OF THE INVENTION

The present invention refers to the medical field. Particularly, it refers to Glycosaminoglycan (GAG), or pharmaceutical composition comprising thereof, for use in a method for the prevention and/or treatment of anaphylaxis; and also to the use of GAG for the diagnosis of anaphylaxis.

### STATE OF THE ART

Anaphylaxis is a systemic hypersensitivity reaction caused for different elicitors that can be life-threatening. The increased vascular permeability and extravasation of fluids associated to the severity of the reactions are hallmarks in its pathophysiology pointing to the endothelium as central. This large organ participates in a multitude of vital functions including coagulation and fibrinolysis modulation, the regulation of immune cells transport, the control of cellular metabolism, or the release of angiocrine mediators. In anaphylaxis, the endothelial layer receives the impact of multiple molecular agents coming from the activation of immune cells and homeostasis imbalance. It impacts in endothelial barrier breakdown which occurs within seconds after activation, resulting in extravasation of vascular contents into the surrounding tissues. In agreement, intravenous fluids administration is a recommended intervention for the acute management of anaphylaxis.

The endothelial cell (EC) surface is covered by a dynamic and shifting structure predominantly composed of glycoproteins and proteoglycans, including glycosaminoglycans (GAGs) side chains, collectively termed endothelial glycocalyx (eGCX). Such malleable sugar architecture acts as signalling platform, essential for the communication between tissues and the blood flow. eGCX controls leukocyte transmigration by endothelial activation of surface receptors (P-selectin, integrins and some immunoglobulins such as cell adhesion molecule 1 (ICAM-1) or vascular adhesion molecule 1 (VCAM-1). Therefore, it is crucial to maintain its structural integrity and keep the endothelial stability by limiting the passage of certain molecules to surrounding tissues and maintaining the homeostasis. Its plasticity makes it highly susceptible to damage, rendering the endothelial membrane unprotected and triggering endothelial dysfunction associated to diseases that include pro-inflammatory conditions as diabetes, hypertension, sepsis, or atherosclerosis. Some of the pathological associated manifestations include capillary leak, augmented migration of proinflammatory cells, and increased endothelial nitric oxide (NO) synthase activity among others. Therefore, strategies aimed at preventing eGCX degradation are potential therapeutic approaches.

eGCX shedding induce local structural alterations that leads to the release of its components into the bloodstream modulating signalling pathways in an endocrine manner. In agreement, circulating eGCX components are potential biomarkers for diagnosing endothelial dysfunction. Among the principal constituents of the eGCX, Endocan, also known as endothelial cell specific molecule-1 (ESM-1), is a soluble dermatan sulfate proteoglycan secreted by vascular ECs. Diverse studies demonstrated that esm-1 mRNA is regulated under the action of diverse molecules such as tumor necrosis factor-α (TNF-α), interleukin-1β (IL-1β), interleukin-8 (IL-8), E-selectin, transforming growth factor-β1 (TGF-β), lipopolysaccharide (LPS), nuclear factor-κB (NF-κB), phorbol myristate acetate (PMA), retinoic acid and vascular endothelial growth factor (VEGF). ESM-1 highlight a crucial role by enhance vascular permeability, leukocyte adhesion and production of proinflammatory cytokines in several diseases. Together with other eGCX components, mainly syndecan-1, heparan sulfate and hyaluronic acid, ESM-1 is a serological biomarker of inflammatory conditions related with eGCX injury. In addition, a higher degradation of such endothelial surface into the bloodstream correlates with disease severity in different pathological settings.

There is an unmet medical need of finding reliable therapeutic strategies aimed at preventing and/or treating anaphylaxis; and biomarkers that give the possibility of diagnosing anaphylaxis. The present invention is focused on solving this problem and an innovative approach is herein provided.

### DESCRIPTION OF THE INVENTION

### Brief description of the invention

The present invention refers to GAGs, or pharmaceutical composition comprising thereof, for use in a method for the prevention and/or treatment of anaphylaxis; and also to the use of GAG for the diagnosis of anaphylaxis.

Particularly, the inventors of the present invention evaluated the structure and function of the eGCX in aortas from active systemic anaphylaxis (ASA) mice by electronic microscopy at only 10 minutes after challenge. WGA staining was used to measure N-acetylglucosamine residues in the vascular wall. A panel of fourteen inflammatory and eGCX-related genes were quantified by RT-PCR. Endocan 1 (ESM-1) protein levels from aorta and sera mice were measured by tissue inmunofluorescence and ELISA. Human sera samples from beta lactams-induced anaphylactic reactions and secretome samples from human microvascular endothelial cells (HMVECs) was used to test ESM-1 levels. Sulodexide (Sdx) was used as a prophylactic treatment in ASA mice.

As a result, ASA mice aortas present reduced surface area and thickness of the eGCX. At that time: ccl2, ccl5, vcam1 and esm1 mRNAs expression increased while ESM-1 protein levels decreased in ASA mice aortas, as well as the WGA content. In addition, ESM-1 sera levels were considerably increased in ASA mice and in the secretome of HMVECs after exposure to acute anaphylactic sera samples. ESM-1 levels were not different between acute and basal samples from anaphylactic patients however, ESM-1 levels are lightly higher than in control individual samples. Furthermore, the use of Sdx increased mice survival by diminishing the severity of the reactions, correlating with the release of ESM-1.

So, in conclusion, the present invention shows that eGCX shedding and specifically ESM-1 appears as a key mediator of ASA mice. Sdx prophylaxis increases the survival of the animals and reduces the appearance of severe reactions. Therefore, it is herein proposed that therapies based in the used of GAGs and proteoglycans could soften these life-threatening reactions.

Although the proof-of-concept provided in the Examples below refers, specifically, to the use of Sdx, which is a purified mixture of glycosaminoglycans composed of heparan sulfate (80%) and dermatan sulfate (20%), for modulating eGCX remodelling, this is a clear indication that GAGs and proteoglycans could be used in the context of the present invention.

### Treatment and/or prevention of anaphylaxis:

So, the first embodiment of the present invention refers to glycosaminoglycan, or any molecule comprising thereof, or pharmaceutical composition comprising thereof, for use in a method for the prevention and/or treatment of anaphylaxis. Alternatively, the present invention refers to a method for the prevention and/or treatment of anaphylaxis which comprises the administration of a therapeutically effective dose of glycosaminoglycan, or any molecule comprising thereof, or pharmaceutical composition comprising thereof.

In a preferred embodiment, the glycosaminoglycan is selected from the group comprising: Hyaluronic Acid (Hyaluronan), chondroitin sulfates, dermatan sulfate, heparan sulfate, heparin, keratan sulfate, and / or mixtures thereof.

In a preferred embodiment, the glycosaminoglycan is characterized by comprising a mixture of heparan sulfate and dermatan sulfate (Sdx).

In a preferred embodiment, the glycosaminoglycan is attached to a protein forming a proteoglycan which comprises a core protein covalently attached which glycosaminoglycan.

In a preferred embodiment, the proteoglycan is selected from the group comprising: Aggregating proteoglycans, small Leucine-Rich Proteoglycans (SLRPs), syndecans, glypicans, perlecan, versican, neurocan, brevican and/or serglycin.

### Diagnosis and/or prognosis of anaphylaxis:

The second embodiment of the present invention refer to an *in vitro* method for the diagnosis and/or prognosis of anaphylaxis, the method comprising assessing the level of glycosaminoglycan, or of any molecule comprising thereof, in a biological sample obtained from the subject.

The third embodiment of the present invention refer to an *in vitro* use of glycosaminoglycan, or of any molecule comprising thereof, for the diagnosis and/or prognosis of anaphylaxis.

In a preferred embodiment, an increased level of glycosaminoglycan in serum samples or a decreased level of glycosaminoglycan in blood vessels or artery tissue, preferably aortic tissue, with respect to a pre-established control level determined in subject who do not suffer from anaphylaxis, is an indication that the subject is suffering or may suffer from anaphylaxis.

In a preferred embodiment, the glycosaminoglycan is selected from the group comprising: Hyaluronic Acid (Hyaluronan), chondroitin sulfates, dermatan sulfate, heparan sulfate, heparin, keratan sulfate, and / or mixtures thereof.

In a preferred embodiment, the glycosaminoglycan is attached to a protein forming a proteoglycan which comprises a core protein covalently attached which glycosaminoglycan.

In a preferred embodiment, the proteoglycan is selected from the group comprising: Aggregating proteoglycans, small Leucine-Rich Proteoglycans (SLRPs), syndecans, glypicans, perlecan, versican, neurocan, brevican and/or serglycin.

In a preferred embodiment, the proteoglycan is endocan (ESM-1).

In a particularly preferred embodiment, the GAG is selected from:
- Hyaluronic Acid (Hyaluronan): Structure: Composed of repeating units of N-acetylglucosamine and glucuronic acid. Characteristics: Does not contain sulfate groups and is not covalently attached to proteins. Functions: Provides lubrication and shock absorption in joints; contributes to tissue hydration and cell migration. Locations: Found in synovial fluid, vitreous humor of the eye, and connective tissues.
- Chondroitin Sulfates: Structure: Made of repeating units of N-acetylgalactosamine and glucuronic acid, with sulfate groups that can vary in position. Characteristics: Highly sulfated, contributing to its negative charge and ability to attract water. Functions: Provides resistance to compression in cartilage and contributes to the structural integrity of tissues. Locations: Predominantly found in cartilage, bone, and skin.
- Dermatan Sulfate: Structure: Composed of repeating units of N-acetylgalactosamine and iduronic acid or glucuronic acid, often with sulfate groups. Functions: Involved in wound repair, coagulation, and cardiovascular functions. Locations: Present in the skin, blood vessels, and heart valves.
- Heparan Sulfate: Structure: Similar to heparin but with a different pattern of sulfation. Composed of glucosamine and either glucuronic or iduronic acid. Functions: Plays a role in cell signaling, regulation of growth factors, and anticoagulation. Locations: Found on cell surfaces and in the basement membrane.
- Heparin: Structure: Highly sulfated and similar to heparan sulfate but with a higher degree of sulfation. Functions: Primarily functions as an anticoagulant by preventing blood clot formation. Locations: Produced by mast cells and stored in granules, released during injury or inflammation.
- Keratan Sulfate: Structure: Contains repeating units of N-acetylglucosamine and galactose, with varying degrees of sulfation. Functions: Provides structural support and contributes to the transparency of the cornea. Locations: Found in cartilage, cornea, and intervertebral discs.

In a particularly preferred embodiment, the proteoglycan is selected from:
- Large, Aggregating Proteoglycans (e.g., Aggrecan). Description: These are large proteoglycans that can aggregate with hyaluronic acid and link proteins to form massive complexes. Functions: Provide structural integrity and resistance to compression, particularly in cartilage. Locations: Primarily found in cartilage, contributing to its load-bearing properties.
- Small Leucine-Rich Proteoglycans (SLRPs). Examples: Decorin, Biglycan, Lumican, and Fibromodulin. Description: Characterized by leucine-rich repeats that help in protein-protein interactions. Functions: Regulate collagen fibril assembly, modulate cell growth, and play roles in tissue repair. Locations: Found in connective tissues, skin, tendons, and the cornea.
- Syndecans: Description: A family of membrane-bound proteoglycans with heparan sulfate and sometimes chondroitin sulfate chains. Functions: Act as co-receptors for growth factors, mediate cell adhesion, and play roles in wound healing and cell signaling. Locations: Present on the surface of many cell types, including epithelial and endothelial cells.
- Glypicans: Description: Proteoglycans attached to the cell membrane through a glycosylphosphatidylinositol (GPI) anchor. They primarily contain heparan sulfate chains. Functions: Involved in modulating signaling pathways like Wnt and Hedgehog, influencing cell growth and development. Locations: Found on the surface of various cell types, particularly in developing tissues.
- Perlecan: Description: A large heparan sulfate proteoglycan present in the basement membrane and extracellular matrix. Functions: Contributes to cell adhesion, filtration in the kidney glomerulus, and the organization of the extracellular matrix. Locations: Found in the basement membrane, cartilage, and other extracellular matrices.
- Versican: Description: A large chondroitin sulfate proteoglycan that contributes to the elasticity and structural integrity of tissues. Functions: Participates in cell adhesion, migration, and proliferation; modulates inflammation and tissue remodeling. Locations: Found in blood vessels, skin, and other connective tissues.
- Neurocan and Brevican: Description: Proteoglycans primarily found in the nervous system. Functions: Play roles in neural development and plasticity, cell adhesion, and inhibition of nerve regeneration after injury. Locations: Located in the brain and spinal cord.
- Serglycin: Description: A proteoglycan associated with secretory granules in cells such as mast cells and cytotoxic T lymphocytes. Functions: Helps package and store proteases and other molecules within secretory granules. Locations: Found in immune cells like mast cells, where it aids in the storage and release of bioactive molecules.

For the purpose of the present invention the following terms are defined:
- The term "comprising" means including, but not limited to, whatever follows the word "comprising". Thus, use of the term "comprising" indicates that the listed elements are required or mandatory, but that other elements are optional and may or may not be present.
- The term "consisting of' means including, and limited to, whatever follows the phrase "consisting of". Thus, the phrase "consisting of" indicates that the listed elements are required or mandatory, and that no other elements may be present.
- By "therapeutically effective dose or amount" of a composition comprising GAGs is intended an amount that, when administered as described herein, brings about a positive therapeutic response in a subject having anaphylaxis or that may have anaphylaxis. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.

### Description of the figures

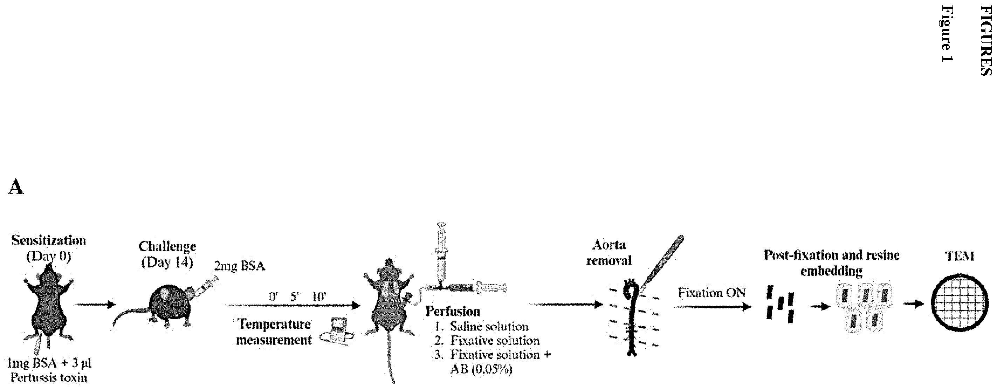

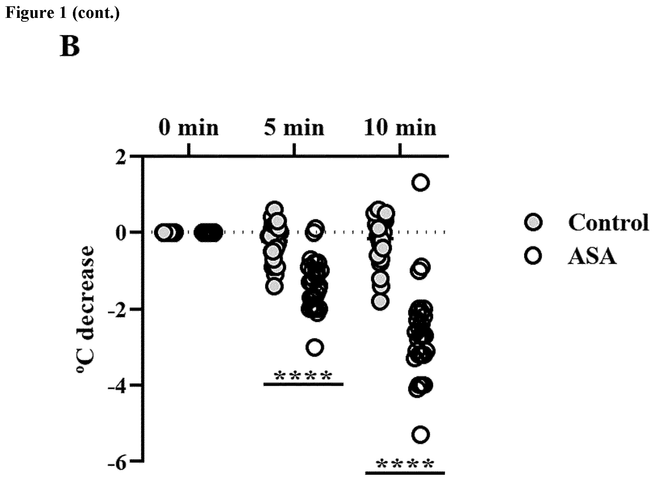

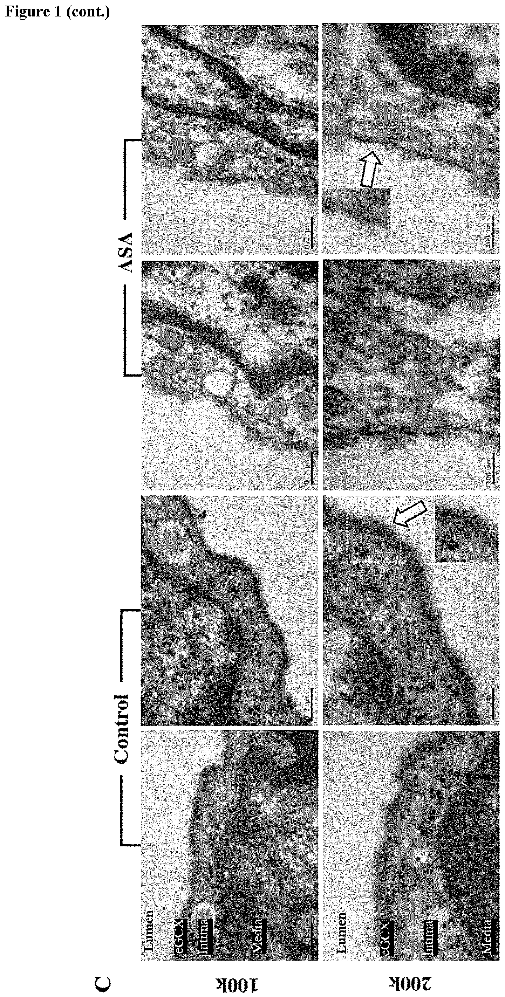

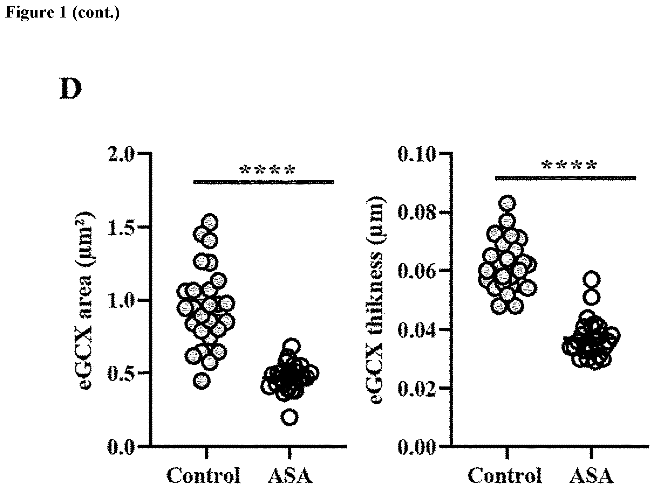

**Figure 1****. Anaphylaxis triggers endothelial glycocalyx shedding in an ASA mice model. A.** Schematic representation of ASA model and TEM procedure. **B.** Rectal temperature recording along the time represented as degree of decrease (n= 26 (control); n=27 (ASA); Sidak's multiple comparison test (****p<0.0001). **C.** Representative TEM images of aortas from 4 control mice and 4 ASA mice at different magnification. Yellow narrows point to eGCX. **D.** Quantification of eGCX surface area (µm2) and thickness (µm) in control (n=4) and ASA(n=4) mice aortas. Shapiro-Wilk test; unpaired t-test and Mann-Whitney test (****p<0.0001). BSA: Bovine Serum Albumin, AB: Alcian's Blue, ON: Overnight, TEM: Transmission Electron Microscopy, ASA: Active Systemic Anaphylaxis, eGCX: endothelial glycocalyx.

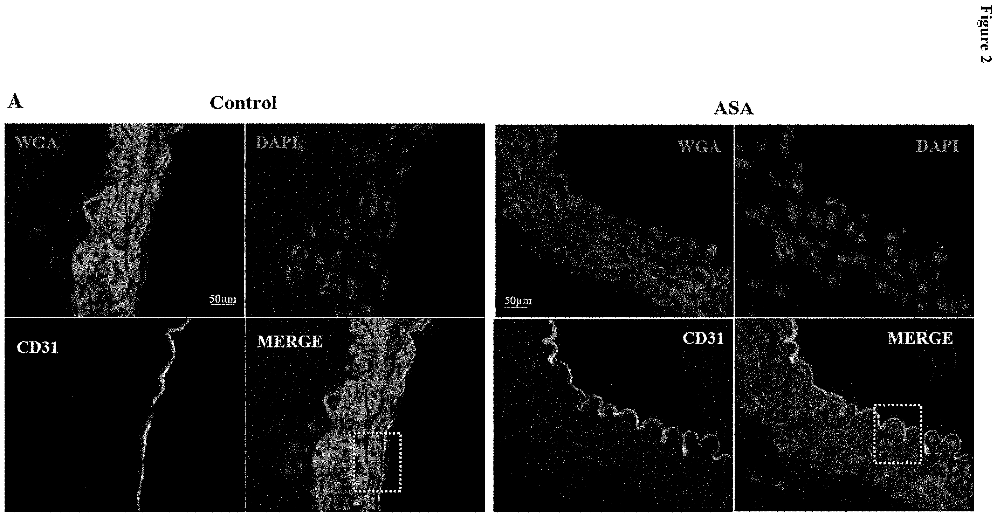

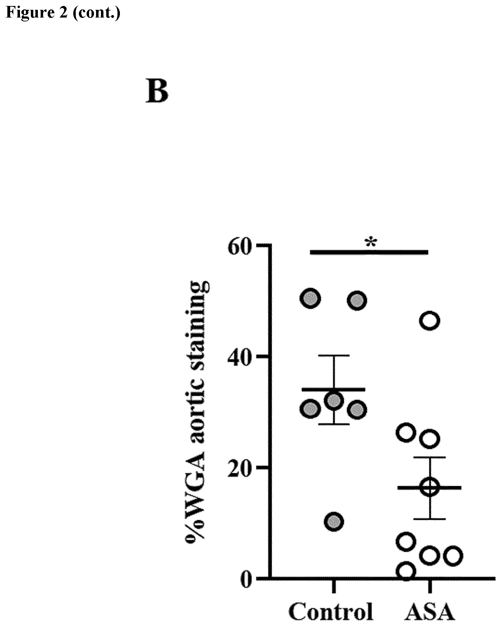

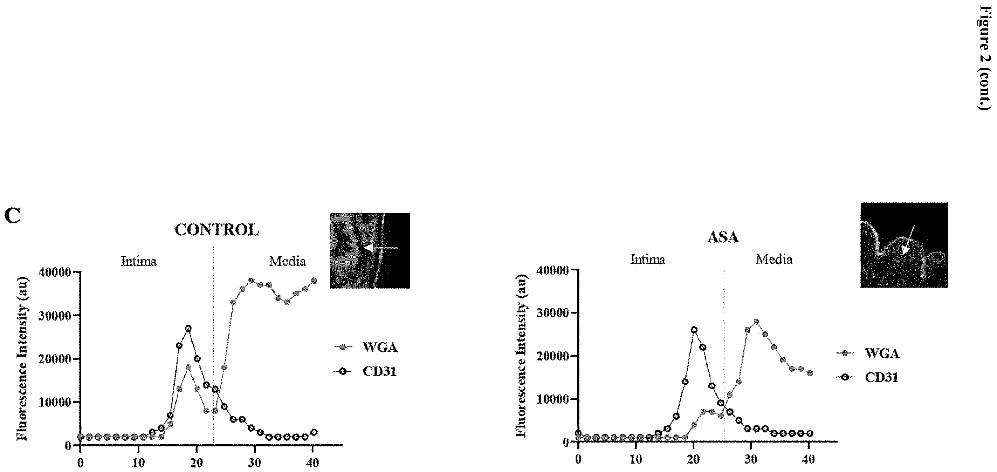

**Figure 2****.** eGCX **proteoglycan components decrease in aortas of ASA mice. A.** Representative images of WGA (red) and CD31 (white) staining in aortic samples. **B.** Quantification of positive WGA staining per area in entire aortic walls from control (n=6) and ASA mice (n=8). Unpaired t-test (*p=0.0544). **C.** Magnification used to quantify WGA intensity profile histogram in the intima and media layer of a representative aortic image from control and ASA mice. WGA: Wheat Germ Agglutinin, ASA: Active Systemic Anaphylaxis.

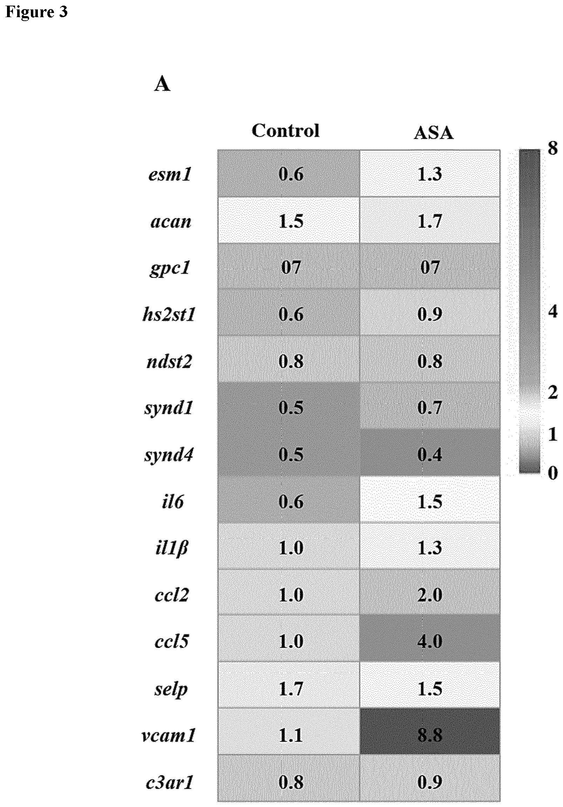

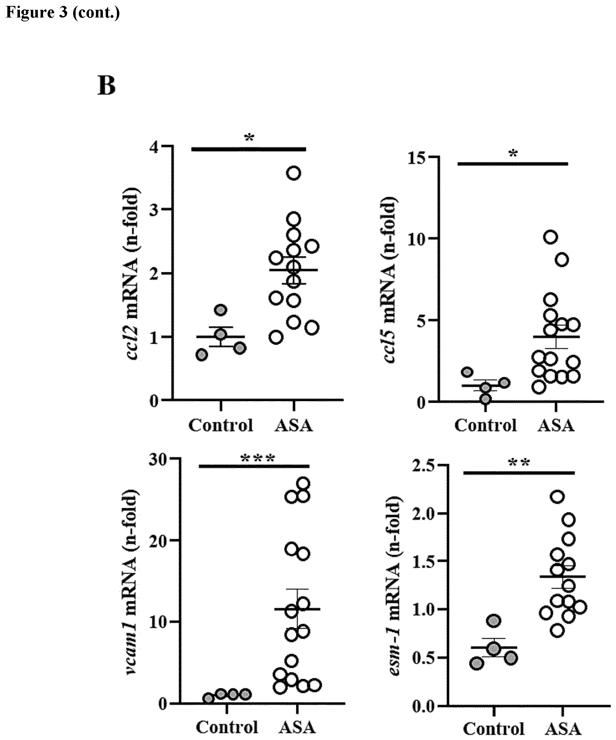

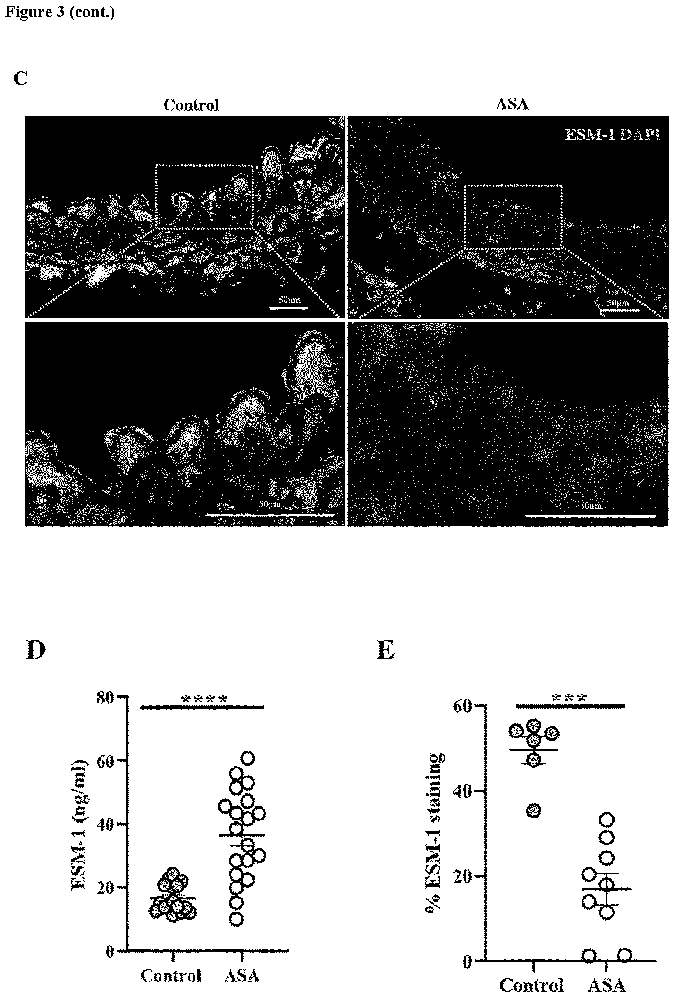

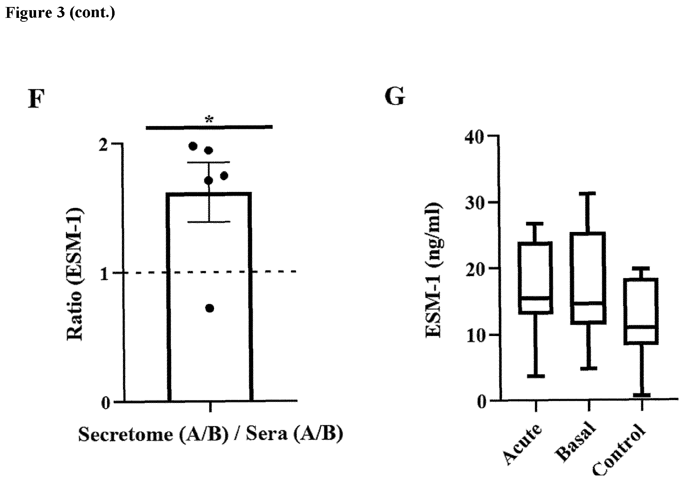

**Figure 3****. ESM-1 is released from aortic tissue and increased in sera of ASA mice. A.** Single heatmap for visualizing data of inflammatory and eGCX genes. Numbers represent medium value of gene expression in aortas from control (n=4) and ASA (n=15) mice. Warm and cold colours relate to high and low levels of gene expression respectively. **B.** Graph shows the quantification of *ccl2, ccl5, vcaml* and *esm-1* RNA expression. Shapiro-Wilk test, unpaired t-test (**p<0.01). C. Representative images of ESM-1 staining in aortic samples of control and ASA mice. **D.** Serum levels of ESM-1 in control (n=16) and ASA (n=19) mice. Shapiro-Wilk test, Mann-Whitney test (****p<0.0001). **E.** Percentage of ESM-1 positive staining per area, in entire aortic layer (media layer and endothelial layer) from control (n=6) and ASA (n=9) mice. Shapiro-Wilk test; Mann-Whitney test (***p<0.001). **F.** Increased ratio of ESM-1 levels in the secretome of serum of anaphylactic patients relative to their baseline levels after endothelial contact (n=5; paired t-test (*p=0.05). **G.** ESM-1 levels in sera from 17 anaphylactic patient's (acute and basal) and 9 control subjects. Comparison of raw ESM-1 values (ng/ml). One way ANOVA and Dunnett's multiple comparisons test (Control vs. Acute: ns=0,197; Control vs. Basal: ns=0,1292). esm1: endothelial specific molecule 1, acan: aggrecan, gpc1: glypican 1, hs2st1: Heparan Sulfate 2-O-Sulfotransferase 1, ndst2: N-Deacetylase And N-Sulfotransferase 2, synd1: syndecan 1, synd4: syndecan 4, ccl2: chemokine (C-C motif) ligand 2, ccl5: chemokine (C-C motif) ligand 5, vcam1; vascular cell adhesion protein 1, il6: Interleukin 6 and il1β: Interleukin 1 β, ASA: Active Systemic Anaphylaxis.

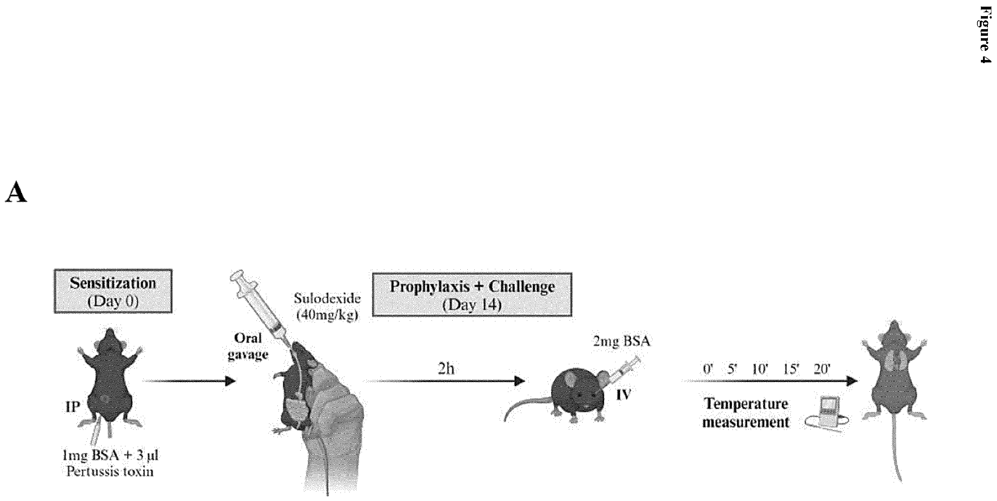

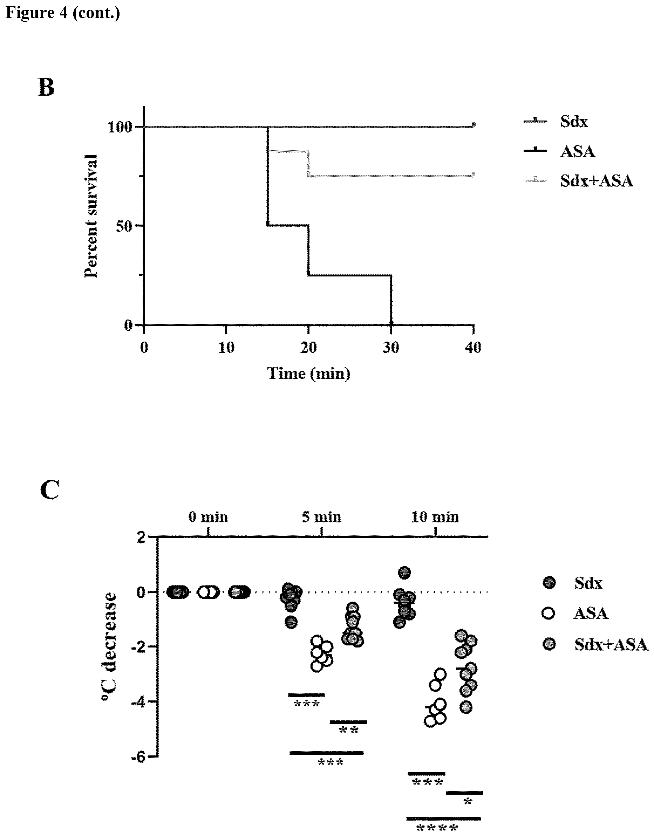

**Figure 4****. Sulodexide prophylactic treatment improves survival and attenuate the severity of anaphylaxis. A.** Schematic representation of ASA model including Sdx prophylaxis. **B.** Survival curve. **C.** Rectal temperature recording after challenge represented as degree of decrease Sdx (n=8), ASA (n=8) ASA+Sdx (n=13). 2way ANOVA with Turkey's multiple comparisons test (ns=not significant; *=p<0.05; **=p<0.01; ***=p<0.001). BSA: Bovine Serum Albumin, IV: Intravenous, Sdx: Sulodexide, WGA: Wheat Germ Agglutinin.

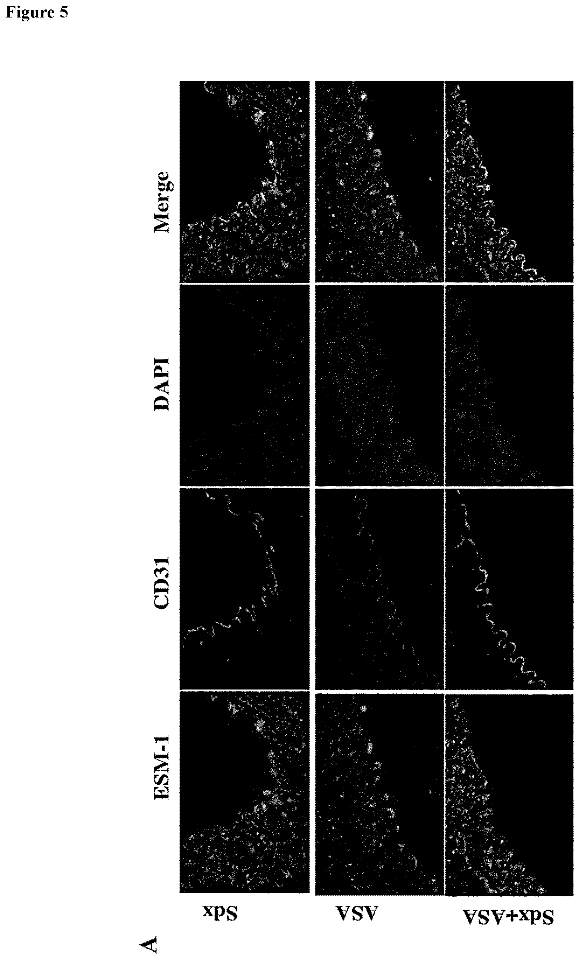

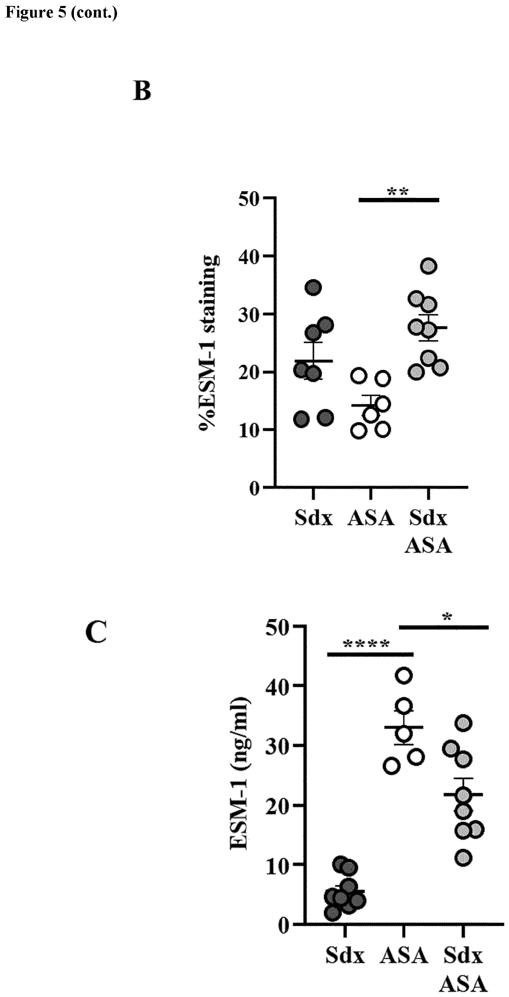

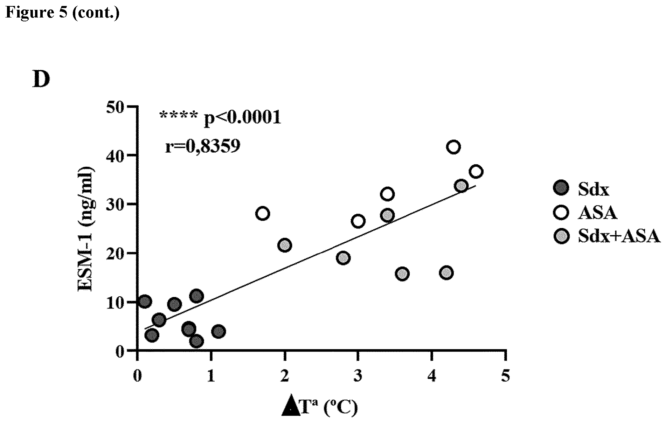

**Figure 5****. Sulodexide prophylactic treatment increases ESM-1 expression in aorta and sera from ASA mice. A.** Representative images of ESM-1 immunofluorescence in aortic samples. **B.** Quantification of positive ESM-1 staining per area in entire aorta. **C.** Serum levels of ESM-1 from Sdx (n=5), ASA mice (n=4) and ASA+Sdx mice (n=6). One way ANOVA with Turkey's multiple comparisons test (*=p<0.05; **=p<0.01; ***=p<0.001). **D.** Correlation between the number of degrees decrease in temperature and ESM-1 amount released to sera. Pearson ESM1: Endothelial Specific Molecule 1.

### Detailed description of the invention

The present invention is illustrated by means of the Examples set below without the intention of limiting its scope of protection.

### Example 1. Material and methods

### Example 1.1. Animal experimental design

Animal procedures were carried out in accordance with European Union Directive 2010/63/EU and Recommendation 2007/526/EC for the care and experimental use of animals. The experimental protocols used in the study, identified by the reference code PROEX: 074.4/21, were approved by the IIS-Fundación Jiménez Diaz (IIS-FJD) Ethics Committee and relevant authorities' forehead. The study involved 8-12-week-old mice of the C57BL/6 strain (Charles Rivers Laboratories) and a model of Active Systemic Anaphylaxis (ASA). Challenged not sensitized mice were included as control of the study and the onset of the anaphylactic reaction was evaluated by monitoring the body temperature of the mice by rectal temperatures before challenge and at five-minute intervals following the reaction using a digital thermometer (VWR 160-200). Two hours before challenge, Sdx (AlfaSigma) (40 mg/kg) was administered by oral gavage in some group of mice. Ten minutes (min) after challenge, mice were euthanized with CO₂, and sera/tissue samples were collected or processed for subsequent molecular analysis.

### Example 1.2. Electron microscopy

eGCX degradation was assessed in aortas of control and ASA mice by transmission electron microscopy (TEM)After saline solution perfusion, mice were followed by a fixative solution containing 2.5% paraformaldehyde (PFA) (Electron Microscopy Science), 2.5% glutaraldehyde (Electron Microscopy Science) and 30mM Magnesium chloride (MgCl2, Sigma). The perfusion was completed with a solution consisting of the previous one plus 0.05% Alcian's Blue (AB) (Sigma), which provides specific staining to acidic polysaccharides such as glycosaminoglycans. After perfusion, the aortas were isolated and divided into 3-4mm fragments, which were immerse in the fixative solution containing 0,05% AB at 4°C during overnight (ON). The following day, the fragments were washed in saline solution and post-fixed with 1% osmium tetroxide (Electron Microscopy Science) and 1% lanthanum tetroxide (Sigma Aldrich) for 1h at room temperature (RT). After washing again in saline solution, the samples were dehydrated in increasing concentrations of ethanol (30%, 50%, 70%, 80%, 90%, 95%, 100%) for 15 minutes at RT each. Then, aortas were gradually embedded in Spurr resin by immersing them in solutions with increasing resin concentration in 100% ethanol (3:1, 1:1, 1:1, 1;3 and pure resin) for 1h at RT each. Aortas were left in pure resin at RT ON and slides were observed under the electron microscopy in Chemistry Faculty of the Universidad Complutense de Madrid. To quantify eGCX, ImageJ software was used. Both the thickness of the eGCX in different areas of the slide and the total area of the covered glycocalyx were quantified at 100K. To measure thickness, six different sections of each animal were evaluated, in turn 6 measurements per image were quantified to obtain a media value per mice.

### Example 1.3. Tissue staining and immunofluorescence

Aortas obtained from control and ASA mice were embedded in paraffin, cut into 4µm thick sections, deparaffinized, and rehydrated before antigenic unmasking was performed using an express pot and citrate buffer (pH 6). To prevent non-specific binding, blocking was done using phosphate buffer (PBS) with BSA 5%, goat serum 10%, and mouse Fc Block (1:100, BD Pharmingen). Anti- ESM-1 primary and anti-CD31 antibodies were used at 1:50 (Abcam). Donkey anti rabbit Alexa Fluor 488 (Invitrogen) was used to detection. Staining with fluorescent Alexa Fluor 555 Wheat Germ Agglutinin (WGA) (Invitrogen) was carried out at dilution 1:2000. Computer-assisted images analysis was performed with Image J software (version 1.0 for Windows). The threshold setting for area measurement was equal for all images. Samples from each animal were examined in a blinded manner. Results were expressed as % positive area vs medial area (WGA, endocan staining). For WGA intensity histograms, Zen software was used. Intensity (au) was measure along a spatial line, drawn from the lumen to the media layer of the artery. The limit between intima a media layer is defined by CD31 staining (endothelium).

### Example 1.4. RNA extraction, RT and qPCR

RNA from aortas was extracted using Tri-Reagent (Molecular Research Center). Two micrograms of total RNA were reverse transcribed according to the high-capacity cDNA Reverse Transcription Kit (Applied Biosystem) protocol. Then samples were stored at -20°C. Quantification of *esm1, synd1, synd4, acan, gpc1, hs2st1, ndst2, c3ar1 and selp* expression was performed with SYBR Green detection method. All primers were purchased from Eurofins Genomics according to the following sequences.
- *esm1* (forward primer (fw): 5' CTG GAG CGC CAA ATA TGC G 3') (SEQ ID NO:1).
- reverse primer (rv): 5' TGAGAC TGT ACG GTA GCA GGT 3') (SEQ ID NO:2).
- *synd1* (fw: 5'CCT TGT CAG GGT AGA CAG CCT 3' (SEQ ID NO:3).
- rv: 5' GAC AGA GGT AAA AGC AGT CTC G 3') (SEQ ID NO:4).
- *synd4* (fw: 5' CAT CTT TGA GAG AAC TGA GGT CTT 3'(SEQ ID NO:5).
- rv: 5' CCT TCT TCT TCA TGC GGT ACA 3') (SEQ ID NO:6).
- *acan* (fw: 5' CGC CAC TTT CAT GAC CGA GA 3' (SEQ ID NO:7).
- rv: 5' TCA TTC AGA CCG ATC CAC TGG TAG 3') (SEQ ID NO:8).
- *gpc1* (fw: 5' GGA GAG CGC ACT CCA TGA C 3') (SEQ ID NO:9).
- rv: 5' CTC AGC ATA TAC GTC CCG GAA 3') (SEQ ID NO:10).
- *hs2st1* (fw: 5' TAT GAT GCC GCC CAA GTT G 3') (SEQ ID NO:11).
- rv: 5' CTG TTC AAT TTC TCG GAC TTC GT 3') (SEQ ID NO:12).
- *ndst2* (fw: 5' CTG CTG ATT GGT TTC AGT CTT 3') (SEQ ID NO:13).
- rv: 5' CCA CTG CTA CTA CAG TCT CCC 3') (SEQ ID NO:14).
- *c3ar1* (fw: 5' TCG ATG CTG ACA CCA ATT CAA3') (SEQ ID NO:15).
- rv: 5' TCC CAA TAG ACA AGT GAG ACC AA 3') (SEQ ID NO:16).
- *selp. 5'-GAA AGG GCT GAT TGT GAC CCC-3' (forward)* (SEQ ID NO:17).
- *5'-AGTAGT TCC GCA CTG GGTACA-3' (reverse)* (SEQ ID NO: 18).

18S rRNA (fw: 5' CCG TCG TAG TTC CGA CCA TAA 3') (SEQ ID NO:19); rv: 5' CAG CTT TGC AAC CAT ACT CCC 3') (SEQ ID NO:20) was used as an internal control. Reactions were incubated for 2min at 50°C followed by 5min at 95°C. Then they were run over 40 amplification cycles (95°C for 15s, 60°C for 1 min and 72°C for 40s) followed by a dissociation stage (95°C for 15s, 60°C for 20s and 98°C for 15s). The quantification of *il1β* (Mm00434228_m1), *il6* (Mm00446190_m1), *ccl2* (Mm00441242_m1), *ccl5* (Mm01302427_m1), *vcam1* (Mm01320970_m1) and 18s rRNA (4310893E) was performed with TaqMan Gene expression assays from Applied Biosystems. Reactions were incubated 20s at 95°C and then run over 40 amplification cycles (95°C for 3s and 60°C for 30s). Double delta Ct analysis was performed in all datasets and heatmaps were generated by using R program with a specific guideline given by "Bioinformatics for all".

### Example 1.5. Anaphylactic Serum samples recruitment and collection

Paired acute and basal serum samples from 17 patients with anaphylaxis to beta-lactams and 9 control samples were used. Following the criteria established by the National Institute of Allergy and Infectious Disease and the Food Allergy and Anaphylaxis Network, 11 of the patients suffered grade 2 reactions, and 6 patients suffered grade 3 reactions accordingly to the severity grading. For serum collection, the blood samples were centrifuged at 1200g for 10 minutes at 4°C. Serum aliquots were stored at -80°C until use. The diagnosis, selection and blood collection of all individuals was carried out in the Allergy and Emergency Departments of Fundación Jiménez Diaz University Hospital and Cruz Roja Central Hospital. Sample collection was always proceeded as fast as possible prioritizing the life of the patients at risk and carrying out the appropriate protocols and drug administration when necessary. The entire project was carried out in accordance with the ethical protocols approved by the Clinical Trials Committee (CEIC-FJD, PIC057-19 and PIC166-22_FJD) and the samples were always collected after informed consent signed by the donors or their families.

### Example 1.6. In vitro human endothelial/sera system

Human Microvascular Dermal Endothelial Cells (HMDEC) were acquired from Lonza (CC-2543). The primary cell culture was grown and expanded with EGM medium (EGTTM-2MV Bullet KitTM Medium) supplemented with: Fetal bovine serum (FBS), hydrocortisone, Human Basic Fibroblast Growth Factor (hFGF-B), Vascular Endothelial Growth Factor (VEGF), recombinant insulin-like growth factor-I analog with the substitution of Arginine for Glutamine at position 3 (R3-IGF-1), ascorbic acid, Human Epidermal Growth Factor (hEGF) and Gentamicin sulfate/Amphotericin (GA-1000) (Lonza). In addition, the medium was also enriched with, heparin (10µl/ml) and EGCF (10µl/ml).

HMDEC were seeded on P6 plates (Corning) and were incubated with patient' sera (both in acute and basal phases independently), at 1: 1 concentration with EGM medium without supplements. The cellular/sera systems were maintained for 2h to mimic an *in vitro* microenvironment that allow the release of soluble mediators from HMDEC in response to sera. Supernatants including the cellular secretome were collected 2 hours of incubation as post-contact, aliquoted and stored at -20°C to measure properly ESM-1.

### Example 1.7. ESM-1 ELISA

A sandwich human ESM-1 ELISA (Elabscience) was used to quantify the abundance of ESM-1 protein in sera from anaphylactic patients and secretomes from HMDEC incubated with patient' sera. Serum samples were quantified at 1: 1 dilution and the secretome directly. Colorimetric detection was performed with the Infinite F200 system (TECAN) following the manufacturer's instructions at a wavelength of 450nm.

A sandwich mice ESM-1 ELISA (Cusabio) was employed to measure the ESM-1 protein levels in sera of mice experiencing anaphylaxis. The serum samples were diluted at a ratio of 1:25 and the colorimetric detection was carried out using the Infinite F200 system from TECAM. The procedure followed the manufacturer's instructions, and the detection was performed at a wavelength of 450nm.

### Example 1.8. Statistical analysis

Data are expressed as mean ± the standard error of the mean (SEM) and the statistical significance of the differences was evaluated with Student's test, Mann Whitney and the analysis of variance (ANOVA) test followed by Tukey's post-hoc or Sidak for multiple comparations, as appropriate. Significance was accepted at the level of p<0.05. Data analysis was performed using GraphPad Prism 8.0 software (GraphPad, San Diego, CA).

### Example 2. Results

### Example 2.1. Anaphylaxis induces endothelial glycocalyx disruption

To evaluate possible eGCX alterations in anaphylaxis, we examined aortic endothelium of an *in vivo* ASA mice model **(****Figure 1A****).** Body temperature was continuously monitored as a physiological marker of the anaphylactic shock, observing that ASA mice exhibited a significant reduction in their body temperature, approximately 3 degrees, within 5-10 minutes post-challenge **(****Figure 1B****).** Next, a comprehensive TEM analysis of the aortic eGCX architecture was conducted. Representative eGCX images of control mice revealed dense ciliary structures uniformly covering the endothelial surface, whereas these structures were markedly reduced or irregular in aortas from ASA mice **(****Figure 1C****).** Quantitatively, a significant reduction of eGCX surface area (0,47 µm²) and thickness (40 nm) was observed in aortic eGCX of ASA mice compared with control mice (0,95 µm² and 60 nm), supporting the loss of eGCX integrity in anaphylaxis **(****Figure 1D****).** Accordingly, WGA staining revealed a significant reduction in glycoproteins content in the whole aortic tissue **(****Figure 2A-B****)** and most specifically in the endothelial layer **(****Figure 2C****)** of ASA mice.

### Example 2.2. Endothelial cell specific molecule-1 is released to sera in anaphylaxis

Next, we evaluated a specific inflammatory/eGCX profile at transcriptomic level in total aortic homogenates **(****Figure 3A****).** Tissues from ASA mice exhibited a significant increased mRNA expression levels of *ccl2, ccl5, vcam1,* and *esm-1* compared to those aortas from control mice **(****Figure 3B**). A certain tendency to increased *il6* and *il1β* mRNA was also observed in ASA mice. However, the expression of *c3ar1, selp, acan*, *sydn-1, sydn-4, ndst2, gpc1*, and *hs2st,* remained unaffected at this time **(Figure Sup1**). Focusing on ESM-1, protein staining was performed revealing a decreased abundance throughout the aortic wall in ASA mice, (control 49% vs ASA 17%), being particularly absent across the endothelial layer (arrows) **(****Figure 3C** and **Figure 3E**). Likewise, ESM-1 serum levels from ASA mice were increased after 10 min of challenge **(****Figure 3D****),** supporting that ESM-1 is regulated and eGCX released from vessels during anaphylaxis. To check ESM-1 relevance in a human system, HMDECs were incubated with serum samples from acute or basal phases of patients with anaphylaxis. Following exposure to acute or basal sera during 2 hours of contact, the secretome of HMDECs showed a substantial increase in ESM-1 protein levels providing compelling evidence that acute anaphylactic sera release ESM-1 from the eGCX **(****Figure 3F****).** Next, to ascertain the potential of ESM-1 as a biomarker in anaphylaxis, circulating ESM-1 levels were evaluated in paired acute and basal sera samples from anaphylactic patients and control subjects. Clinical characteristics of the patient cohort are summarized in **Table 1.**

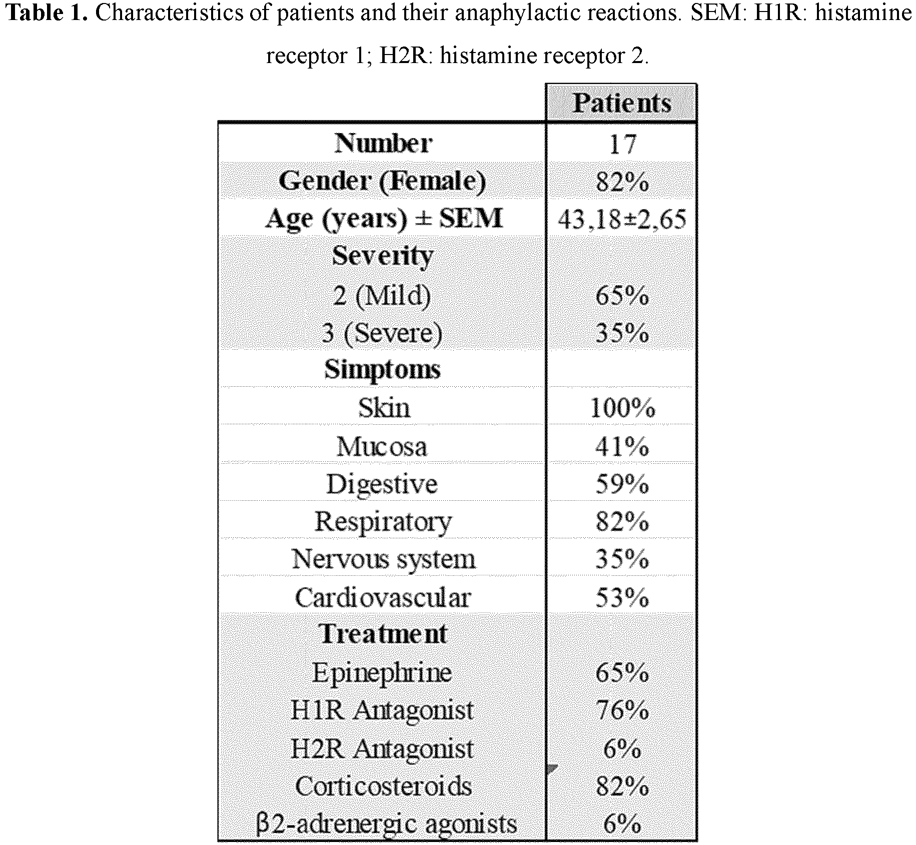

No significant differences in ESM-1 levels were seen between samples collected during the acute phase or basal state from individuals that suffer an anaphylactic reaction. However, a rise tendency was observed in anaphylactic patients when compared with sera from control subjects **(****Figure 3G****).**

### Example 2.3. Sulodexide prophylaxis prevents eGCX disruption, drop of body temperature and ESM-1 release in ASA mice

To investigate the impact of eGCX shedding prevention in anaphylaxis, sensitized mice were prophylactically treated with Sdx two hours before challenges **(****Figure 4A****).** The survival rate was notably improved in Sdx+ASA mice **(****Figure 4B****).** In agreement, continuous body temperature monitoring indicated that ASA challenge induced a reduction of 2 degrees at 5 minutes and 4 degrees at 10 minutes post-challenge while Sdx administration significantly attenuated the fall in temperature in Sdx+ASA mice **(****Figure 4C****).** WGA staining shown that Sdx tends to decrease proteoglycan content within the vessel wall induced by anaphylactic shock **(Sup 2).** Moreover, protein levels of ESM-1 are regulated by Sdx treatment, exhibiting an increased staining in aortas from Sdx+ASA mice **(****Figure 5 A-B****).** Finally, ESM-1 sera levels were decreased in Sdx treated mice **(****Figure 5C****)** and these levels were correlated with decrease in temperature of the mice **(****Figure 5D****).**

## Claims

1. Glycosaminoglycan, or any molecule comprising thereof, or pharmaceutical composition comprising thereof, for use in a method for the prevention and/or treatment of anaphylaxis.

2. Glycosaminoglycan, or any molecule comprising thereof, or pharmaceutical composition comprising thereof, for use, according to claim 1, selected from the group comprising: Hyaluronic Acid (Hyaluronan), chondroitin sulfates, dermatan sulfate, heparan sulfate, heparin, keratan sulfate, and / or mixtures thereof.

3. Glycosaminoglycan, or any molecule comprising thereof, or pharmaceutical composition comprising thereof, for use, according to any of the previous claims, **characterized by** comprising a mixture of heparan sulfate and dermatan sulfate.

4. Glycosaminoglycan, or any molecule comprising thereof, or pharmaceutical composition comprising thereof, for use, according to any of the previous claims, **characterized in that** the glycosaminoglycan is attached to a protein forming a proteoglycan which comprises a core protein covalently attached which glycosaminoglycan.

5. Glycosaminoglycan, or any molecule comprising thereof, or pharmaceutical composition comprising thereof, for use, according to any of the previous claims, wherein the proteoglycan is selected from the group comprising: Aggregating proteoglycans, small Leucine-Rich Proteoglycans (SLRPs), syndecans, glypicans, perlecan, versican, neurocan, brevican and/or serglycin.

6. *In vitro* method for the diagnosis and/or prognosis of anaphylaxis, the method comprising assessing the level of glycosaminoglycan, or of any molecule comprising thereof, in a biological sample obtained from the subject.

7. *In vitro* use of glycosaminoglycan, or of any molecule comprising thereof, for the diagnosis and/or prognosis of anaphylaxis.

8. *In vitro* method, or *in vitro* use, according to any of the claims 6 or 7, wherein an increased level of glycosaminoglycan in serum samples or a decreased level of glycosaminoglycan in blood vessels or artery tissue, preferably aortic tissue aortic tissue, with respect to a pre-established control level determined in subject who do not suffer from anaphylaxis, it is an indication that the subject is suffering or may suffer from anaphylaxis.

9. *In vitro* method, or *in vitro* use, according to any of the claims 6 to 8, wherein the glycosaminoglycan is selected from the group comprising: Hyaluronic Acid (Hyaluronan), chondroitin sulfates, dermatan sulfate, heparan sulfate, heparin, keratan sulfate, and / or mixtures thereof.

10. *In vitro* method, or *in vitro* use, according to any of the claims 6 to 9, **characterized in that** the glycosaminoglycan is attached to a protein forming a proteoglycan which comprises a core protein covalently attached which glycosaminoglycan.

11. *In vitro* method, or *in vitro* use, according to any of the claims 6 to 10, wherein the proteoglycan is selected from the group comprising: Aggregating proteoglycans, small Leucine-Rich Proteoglycans (SLRPs), syndecans, glypicans, perlecan, versican, neurocan, brevican and/or serglycin.

12. *In vitro* method, or *in vitro* use, according to any of the claims 6 to 11, wherein the proteoglycan is endocan (ESM-1).
